# EUROPÄISCHE PATENTANMELDUNG

(11) **EP 1 958 931 A1**
(43) Veröffentlichungstag der Anmeldung: **20.08.2008**
(21) Anmeldenummer: 07002256.1
(22) Anmeldetag: 02.02.2007
(51) Int. Cl.: C07C 69/30, C07C 69/52, C10M 105/38

(54) **Oxidationsstabile Carbonsäureester und deren Verwendung**

(71) Anmelder: Cognis IP Management GmbH, 40589 Düsseldorf (DE); Cognis Oleochemicals GmbH, 40589 Düsseldorf (DE)
(72) Erfinder: Westfechtel, Alfred, 40724 Hilden (DE); Hof, Matthias, 47249 Duisburg (DE)
(74) Vertreter: Reinhardt, Jürgen

(57) **Zusammenfassung**

Carbonsäureester, die als Alkoholkomponente Trimethylolpropan und als Säurekomponente eine Mischung aus (i) linearen gesättigten Fettsäuren mit 8 bis 18 C-Atomen und (ii) linearen ein- oder mehrfach ungesättigten Fettsäuren mit 12 bis 22 C-Atomen enthalten, wobei dass das Molverhältnis (i) : (ii) im Bereich von 3 : 1 bis 1: 3 liegt zeigen gute Stabilität gegenüber Oxidation und eignen sich als Grundöle für Hydrauliköle.

## Beschreibung

Die Erfindung betrifft ausgewählte Carbonsäureester und deren Verwendung als Bestandteil von Hydraulikölen oder in Schmiermitteln.

Hydraulische Systeme werden in einer Vielzahl von technischen Geräten, beispielsweise Automobilen, Lastkraftwagen, Kränen, Zügen und anderen Transportmitteln, aber auch bei landwirtschaftlichen Geräten, Schiffen sowie in Industriebetrieben und im Eisenbahnsektor eingesetzt. Die hydraulischen Systeme enthalten in aller Regel eine hydraulische Flüssigkeit, die in traditioneller Weise petrochemischen Flüssigkeiten oder Ester bzw. andere Oleochemikalien als Basisflüssigkeit enthält. Letztere sind aufgrund ihrer biologischen Abbaubarkeit zunehmend bevorzugt.

In diesem Zusammenhang sind insbesondere Polyolester von Fettsäuren, vorzugsweise ungesättigten Fettsäuren aus dem Stand der Technik als geeignete Basisflüssigkeiten für Hydrauliköle bekannt. So offenbart die WO 97/39086 Ester, die durch Umsetzung von Polyolen, darunter auch Trimethylolpropan, mit Mischungen von Fettsäuren erhalten werden, wobei das Verhältnis von kurzkettigen Fettsäuren zu den langkettigen Fettsäuren im Bereich von 2 : 1 bis 1 : 20 liegen muss. Der Schrift ist zu entnehmen, dass die beanspruchten Ester insbesondere aufgrund ihrer Tieftemperaturverträglichkeit vorteilhafte technische Eigenschaften aufweisen sollen. Allerdings setzt die WO 97/39086 Mischungen von Fettsäuren voraus, wobei der kurzkettige Anteil allein aus der Gruppe der C5-C 12 Fettsäuren, in den Beispielen ausschließlich den C8-C10 Fettsäuren ausgewählt wird. Die DE 101 15 829 A1 beschreibt oxidationsstabile Polyolester, die hergestellt werden können, indem man technische Ölsäure als Säurekomponente einsetzt.

Neben der Tieftemperaturstabilität müssen Estersysteme und Hydraulikflüssigkeiten allerdings auch eine hohe Oxidationsstabilität aufweisen, die insbesondere dann zum Tragen kommt, wenn das Hydrauliköl in Gegenwart von Luftsauerstoff hohen Temperaturen ausgesetzt ist. Um dies zu erreichen, werden gemäß dem Stand der Technik Antioxidantien als übliche Additive den Hydraulikölen beigemengt. Maßgeblich ist in diesem Fall der so genannte modifizierte, trockene "Turbine oxidation stability test", kurz trockner TOST nach DIN51587, der die Stabilität von Prüfölen bei Alterung bei 95 °C in Gegenwart von Sauerstoff prüft.

Herkömmliche Systeme erreichen hier bereits nach 170 bis etwa 300 Stunden den kritischen Grenzwert von 2,0 mg KOH/g Prüföl. Da aber zunehmend höhere Oxidationsstabilität gefordert wird, besteht seitens der Industrie ein ständiger Bedarf, oxidationsstabilere Öle bereitstellen zu können. Der Versuch, durch Zusatz weiterer Antioxidantien die gewünschte hohe Oxidationsstabilität zu erreichen, ist bislang noch nicht erfolgreich gewesen.

Es war daher die Aufgabe der vorliegenden Erfindung, oxidationsstabile Flüssigkeiten für den Einsatz in Hydraulikölen bereitzustellen, die auch in Bezug auf ihre biologische Abbaubarkeit den aktuellen Anforderungen entsprechen. Überraschend wurde gefunden, dass ausgewählte Polyolester die obigen Forderungen erfüllen.

Gegenstand der vorliegenden Anmeldung sind daher synthetische Carbonsäureester, die als Alkoholkomponente Trimethylolpropan und als Säurekomponente eine Mischung aus (i) linearen gesättigten Fettsäuren mit 8 bis 18 C-Atomen und (ii) linearen ein- oder mehrfach ungesättigten Fettsäuren mit 12 bis 22 C-Atomen enthält wobei das Molverhältnis (i) : (ii) im Bereich von 3 : 1 bis 1 : 3 und vorzugsweise im Bereich von 2 : 1 bis 1 : 3 und besonders bevorzugt im Bereich von 1 : 1 bis 1 : 3 liegt.

Die erfindungsgemäßen synthetischen Ester werden auf an sich bekannte Art und Weise durch Umsetzung des Trimethylolpropans mit den Mischungen aus gesättigten und ungesättigten Fettsäuren in Gegenwart geeigneter Katalysatoren und bei erhöhter Temperatur hergestellt. Die Reaktionsprodukte können dann destillativ aus dem Ansatz entfernt werden. Es kann vorteilhaft sein, die Reaktion unter einer Inertgasatmosphäre, vorzugsweise Stickstoff, durchzuführen.

Wesentliches technisches Merkmal bei der Herstellung der erfindungsgemäßen Ester ist das Molverhältnis der Fettsäuremischung. Nur in dem beanspruchten, engen Bereich von 3 : 1 bis 1 : 3 bzw. vorzugsweise 2 : 1 bis 1 : 3 und besonders bevorzugt im Bereich von 1 : 1 bis 1 : 3 werden die gewünschten Ester erhalten. Ein weiterer bevorzugter Bereich für das Molverhälnis (i) zu (ii) liegt bei 2 : 1 bis 1 : 2 und hier vorzugsweise bei 1 :1 bis 1 : 2.

Als gesättigte linearen Fettsäuren können solche mit 8 bis 18, vorzugsweise 8 bis 16 und insbesondere 8 bis 14 C-Atome Verwendung finden. Es kommen konkret und vorzugsweise in Betracht: Oktansäure, Perlagonsäure, Nonansäure, Decansäure, Undecansäure, Dodecansäure, Tridecansäure, Tetradecansäure, Pentadecansäure, Palmitinsäure, Margarinsäure, Stearinsäure und Nonandecansäure sowie beliebige Mischungen dieser Säuren untereinander. Vorzugsweise werden Mischungen aus C8- aus C14-Säuren zur Synthese der erfindungsgemäßen Ester eingesetzt. Besonders bevorzugt sind als linearen gesättigten Fettsäuren (i) solche Fettsäuremischungen, die zu mehr als 80 Gew.-%, vorzugsweise mehr als 85 Gew.-% und insbesondere mehr als 90 Gew.-% Fettsäuren der Kettenlänge C-12 bis C-18 und vorzugsweise C-12 bis C-14 enthalten. Bevorzugte Fettsäuremischungen des Typs (i) sind frei von Fettsäuren der Kettenlänge C-18. Nicht umfasst sind auch Säuremischungen aus Oktansäure und Dekansäure im Gewichtsverhältnis von 55 : 45 als Komponente (i). Diese werden in der WO 97/39086 in den Beispielen konkret offenbart.

Als ungesättigte Fettsäure ist die Ölsäure ausgewählt. Diese kann auch in technischer Qualität eingesetzt werden, bevorzugt ist aber eine reine Ölsäure. Ölsäure wird im Wesentlichen aus ihren natürlichen Vorkommen in pflanzlichen und tierischen Fetten und Ölen gewonnen. Dazu werden die Triglyceride zunächst einer Druckspaltung unterworfen und die resultierenden Gemische der verschiedenen Fettsäuren durch das Verfahren der Umnetzung in einen gesättigten Anteil (Stearin) und einen ungesättigten, weitgehend aus Ölsäure bestehenden Anteil (Olein) separiert. Vorzugsweise wird eine gereinigte Ölsäure verwendet. Weniger geeignet ist aber z.B. eine technische Ölsäure, wie sie z.B. durch Druckspaltung und anschließender Umnetztrennung gewonnen wird. Technische Ölsäure enthält typischerweise nur ca. 70 Gew.-% der einfach ungesättigten Ölsäure, wobei die restlichen 30 Gew.-% auch mehrfach ungesättigte Säuren, und ungesättigte Säuren, teilweise C-18 und C-16 entfällt. Die vorliegende Erfindung macht aber vorteilhafter weise und daher bevorzugt Gebrauch von reinen Ölsäurequalitäten, die mehr als 70 %, vorzugsweise mehr als 80 % und insbesondere mehr als 90 % Ölsäure enthalten.

Ausgeschlossen sind aber solche Fettsäuremischungen, wie sie in der oben zitierten DE 101 15 829 A1 in der Spalte 2, Zeile 60 bis Spalte 3, Zeile 9, und im Ausführungsbeispiel dieser Schrift konkret offenbart werden.

Besonders bevorzugt sind solche Ester, bei denen das das molare Verhältnis zwischen der Säurekomponente (als den Säuren des Typs (i) und (ii) zusammen) und dem Trimethylolpropan im Bereich von 5 : 1 bis 1 : 1, vorzugsweise 2 : 1 bis 1 : 1 und besonders bevorzugt 1,5 : 1 bis 1 : 1 beträgt, wobei hier die Verhältnisse bei der Synthese - also die molaren Verhältnisse der Ausgangsverbindungen betrachtet werden.

Ein weiterer Aspekt der vorliegenden Erfindung betrifft den Einsatz der beschriebene Ester als Bestandteile eines Hydrauliköls, und insbesondere als Basis- oder Grundöl darin. Solche Hydrauliköle können dann vorzugsweise bis zu 95 Gew.-% der Ester enthalten wobei aber Mengen zwischen 25 und 85 Gew.-% bevorzugt sind. Diese Öle enthalten neben dem Grundöl in der Regel auch Additive, vorzugsweise in Mengen von 5 bis 25 Gew.-%. Bei den Additiven handelt es sich um die dem Fachmann prinzipiell bekannten Klassen, nämlich Antioxidantien, extrem pressure (EP) bzw. anti-wear (AW) Additiven, Korrosionsinhibitoren, Demulgatoren und/oder Entschäumern. Weiterhin können noch Buntmetalldeaktivatoren enthalten sein.

Die Additive sind in üblichen Mengen, insgesamt aber in max. 10 Gew.-%, vorzugsweise 1 bis 3 Gew.-% bezogen auf das Gesamtgewicht des Hydrauliköls enthalten. Dabei sind die EP/AW Additive in Mengen von vorzugsweise 0,2 bis 2,0 Gew.-%, Antioxidantien im Bereich von 0,2 bis 1,0 Gew.-%, Korrosionsschutzadditive im Bereich von 0,05 bis 0,2 Gew.-%, Buntmetalldeaktivatoren im Bereich von 0,05 bis 0,5 und Antischaumadditive Entschäumer im Bereich von 0,005 bis 0,04 % in den erfindungsgemäßen Hydraulikölen enthalten.

Die Formulierung derartiger Hydrauliköle erfolgt durch Vermischen der Basisflüssigkeit mit den Additiven, ggf. bei erhöhter Temperatur. Die Ester der vorliegenden Anmeldung können auch und bevorzugt als Bestandteil von Schmiermitteln Verwendung finden.

### Beispiele

### 1. Herstellung der erfindungsgemäßen Ester

100 g einer Mischung gesättigter, linearer Fettsäuren des C-Kettenschnitts C8-C14 (0,5 mol) wurden mit 280 g Ölsäure (1,0 mol) sowie 0,45 g eines Katalysators (FASCAT^{®} 2001, Fa. Arkema) vermischt. Diese Mischung wurde zusammen mit 74 g Trimethylolpropan (=TMP) (0,55 mol) für mehrere Stunden auf 240 °C erwärmt. Die Fettsäuremischung der kurzkettigen linearen gesättigten Fettsäuren (i) war wir folgt zusammengesetzt (Angaben in Gew.-%): 7 % C-8, 8 % C-10, 62 % C-12 und 19 % C-14 Fettsäuren. Als Ölsäure wurde eine technische Qualität eingesetzt. Anschließend wurde das Wasser destillativ entfernt Das rohe. Reaktionsprodukt wurde abgekühlt und filtriert. Die Ausbeute betrug 99 % d. Th.

### 2. Herstellung der Vergleichsester

Analog zu der Verfahrensweise wie oben beschrieben, wurden auch die Vergleichsester hergestellt, allerdings wurden andere Gewichtsverhältnisse ausgewählt. Details sind der folgenden Tabelle zu entnehmen. In allen Fällen war TMP die Alkoholkomponente.

**Tabelle 1**

| **Nr.** | **Fettsäure(n)** | **Gew.-Verh.** | **Mol.-Verh.** | **Trübungspunkt [°C]** | **Pour Point [°C]** | **dyn. Viskosität [cps]** | **VZ** | **SZ** | **OHZ** |
|---|---|---|---|---|---|---|---|---|---|
| 1 | C8-C14 | 100 | 100 | -5 | -5 | 34 | 243 | 3,4 | 27 |
| 2 | Ölsäure | 100 | 100 | - 30 | - 40 | 45 | 190 | 0,7 | 15 |
| 3 | C8-C14/ Ölsäure | 78 : 22 | 1 : 0,5 | - 10 | -11 | 40 | 229 | 3,4 | 26 |
| 4 | C8-C14/ Ölsäure | 42: 48 | 1 : 1 | -20 | - 22 | 42 | 206 | 3,3 | 23 |
| 5 | C8-C14/ Ölsäure | 26:74 | 0,5 : 1 | - 24 | - 28 | 40 | 198 | 0,95 | 19 |

Man erkennt, dass der erfindungsgemäß ausgewählten TMP-Ester 3 bis 5 die besten Tieftemperatureigenschaften aufweisen, verglichen mit den Estern 1 und 2.

### 3. Anwendungstechnische Eigenschaften

Zur Prüfung der anwendungstechnischen Eigenschaften der Ester wurden diese gemäß DIN 51587 gestestet (so genannter DRY TOST). Die erfindungsgemäßen Ester 3 bis 5 gemäß Tabelle 1 und der Ester aus Ölsäure mit TMP Nr. 2 wurden jeweils zusammen mit einem Additivpaket, enthaltend u.a. Antioxidantien, Metalldeaktivatoren, Entschäumer etc., untersucht.

Die Säurezahlen in Abhängigkeit von der Zeit wurden gemessen. Ergebnisse der Säurezahldifferenz sind in der folgenden Tabelle wiedergegeben. Die Untersuchung wurde beendet, sobald die Differenz der Säurezahl bei 2 oder mehr lag.

**Tabelle 2**

| **Nr.** | **Fettsäure(n)** | **Start** | **168 h** | **336 h** | **504 h** |
|---|---|---|---|---|---|
| 2 | Ölsäure | 0 | 11,3 | - | - |
| 5 | C8-C14/ Ölsäure | 0 | 0,4 | 0,7 | 10,6 |

Der erfindungsgemäße TMP-Ester Nr. 5 weist eine gegenüber einem reinen Ölsäureester verbesserte Oxidationsstabilität auf.

## Patentansprüche

1. Carbonsäureester, der als Alkoholkomponente Trimethylolpropan und als Säurekomponente eine Mischung aus (i) linearen gesättigten Fettsäuren mit 8 bis 18 C-Atomen und (ii) linearen ein- oder mehrfach ungesättigten Fettsäuren mit 12 bis 22 C-Atomen enthält, **dadurch gekennzeichnet, dass** das Molverhältnis (i) : (ii) im Bereich von 3 : 1 bis 1 : 3 und bevorzugt von 1 : 1 bis 1 : 3 liegt.

2. Carbonsäureester nach Anspruch 1, **dadurch gekennzeichnet, dass** das molare Verhältnis zwischen der Säurekomponente und dem Trimethylolpropan im Bereich von 5 : 1 bis 1 : 1, vorzugsweise 2 : 1 bis 1 : 1 und besonders bevorzugt 1,5 : 1 bis 1 : 1 beträgt.

3. Carbonsäureester nach mindestens einem der Ansprüche 1 und 2, **dadurch gekennzeichnet, dass** als Säurekomponente (i) Fettsäuren mit 8 bis 16, vorzugsweise 8 bis 14 C-Atomen ausgewählt sind.

4. Carbonsäureester nach mindestens einem der Ansprüche 1 bis 3, **dadurch gekennzeichnet, dass** als Säurekomponente (ii) Ölsäure ausgewählt ist.

5. Carbonsäureester nach mindestens einem der Ansprüche 1 bis 4, **dadurch gekennzeichnet, dass** Säuremischungen aus Oktansäure und Dekansäure im Gewichtsverhältnis von 55 : 45 als Komponente (i) ausgeschlossen sind.

6. Carbonsäureester nach mindestens einem der Ansprüche 1 bis 5, **dadurch gekennzeichnet, dass** die linearen gesättigten Fettsäuren (i) Fettsäuremischungen verwendet werden, die zu mehr als 80 %, vorzugsweise mehr als 85 % und insbesondere mehr als 90 % Fettsäuren der Kettenlänge C-12 bis C-18 enthalten.

7. Carbonsäureester nach Anspruch 6, **dadurch gekennzeichnet, dass** als ungesättigte Fettsäure (ii) Ölsäure verwendet wird.

8. Verwendung von Carbonsäureestern nach mindestens einem der Ansprüche 1 bis 7 als Basisflüssigkeit in Hydraulikölen oder in Schmiermitteln.
